# EUROPÄISCHE PATENTANMELDUNG

(11) **EP 1 101 752 A2**
(43) Veröffentlichungstag der Anmeldung: **23.05.2001**
(21) Anmeldenummer: 00123287.5
(22) Anmeldetag: 27.10.2000
(51) Int. Cl.: C07C 29/141, C07C 33/46

(54) **Verfahren zur Herstellung halogenierter Benzylalkohole**

(30) Priorität: 19.11.1999 DE 19955650
(71) Anmelder: Clariant GmbH, 65929 Frankfurt am Main (DE)
(72) Erfinder: Krause, Jochen, Dr., 60322 Frankfurt am Main (DE); Dierdorf, Andreas, Dr., 60529 Frankfurt (DE); Wessel, Thomas, Dr., 61138 Niederdorfelden (DE); Pfirmann, Ralf, Dr., 64347 Griesheim (DE); Degend, Matthias, 63739 Aschaffenburg (DE); Kokott, Sebastian, 65916 Frankfurt am Main (DE)

(57) **Zusammenfassung**

Die vorliegende Erfindung betrifft ein Verfahren zur Herstellung von Verbindungen der Formel (I) 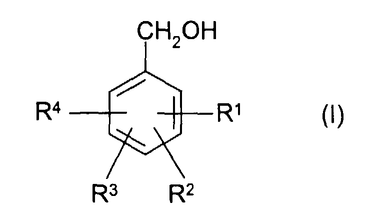 indem man eine Verbindung der Formel (II) 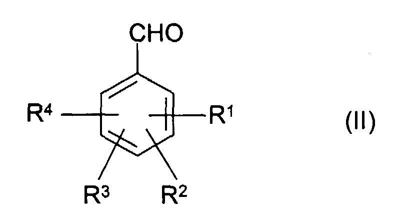 in Gegenwart eines Palladium oder Platin enthaltenden Katalysators, in Anwesenheit oder Abwesenheit eines Lösungsmittels mit Wasserstoff bei 0 bis 120 °C umsetzt, wobei die Reste R¹, R², R³ und R⁴ in der Formel (I) dieselbe Bedeutung wie in Formel (II) haben und R¹ für Cl oder F steht, R², R³ und R⁴ unabhängig voneinander gleich oder verschieden sind und für H, F, Cl, CF₃, OCF₃, C₁-C₄-Alkyl oder C₁-C₄-Alkoxy stehen.

## Beschreibung

Die vorliegende Erfindung betrifft ein vorteilhaftes Verfahren zur Herstellung halogenierter Benzylalkohole durch katalytische Hydrierung entsprechend halogenierter Benzaldehyde.

Halogenierte Benzylalkohole finden eine breite Anwendung als Synthesebausteine zur Herstellung von Vorprodukten für Pharmaka und Pflanzenschutzmittel. Sie können auch unmittelbar als Bestandteile mikrobiozider Mittel eingesetzt werden (DE-OS 23 33 849).

Die Herstellung halogenierter Benzylalkohole erfolgt üblicherweise durch nicht-katalytische Reduktion entsprechender halogenierter Aldehyde, beispielsweise mittels Aluminiumalkoholaten (Meerwein-Ponndorf-Verley-Reaktion), mittels komplexer Hydride wie NaBH₄ oder LiAIH₄, mittels Formaldehyd, mittels Hydrosilylierung, mittels Na-dithionit oder Na-sulfid. Bei diesen nicht-katalytischen Reduktionen fällt das oxidierte Reduktionsmittel als Abfallprodukt an.

Die US 3 527 817 betrifft die katalytische Hydrierung halogenierter aromatischer Ketone und Aldehyde zu den entsprechenden Alkoholen mittels Wasserstoff in Gegenwart von Kupferchromit-Katalysatoren unter Zusatz eines Oxides oder Hydroxides eines Erdalkalimetalls. Die Umsetzung von o-Chlorbenzaldehyd und p-Chlorbenzaldehyd führt zu einem Umwandlungsgrad von 39 % (Table II, Example 10) bzw. von 90,7 % (Table II, Example 11).

Aus Beispiel 1 der DE-OS 23 33 849 geht die katalytische Hydrierung von 2,4-Difluorbenzaldehyd mittels Wasserstoff in Gegenwart von Raney-Nickel als Katalysator hervor. Die Ausbeute an 2,4-Difluorbenzylalkohol beträgt 68,6 % der Theorie.

Die nicht-katalytischen Reduktionsverfahren sind technisch recht aufwendig, erfordern vergleichsweise teure Reduktionsmittel und führen zu Abfallprodukten, die in stöchiometrischen Mengen anfallen und entsorgt werden müssen.

Die vorstehend erwähnten katalytischen Hydrierungen liefern die gewünschten halogenierten Benzylalkohole in zum Teil sehr niedrigen Ausbeuten.

Im Hinblick hierauf besteht der Bedarf, ein Verfahren bereitzustellen, das die vorstehend geschilderten Nachteile vermeidet, sich einfach ausführen läßt und die gewünschten Produkte in hoher Ausbeute und zugleich hoher Reinheit zugänglich macht.

Gelöst wird die Aufgabe durch ein Verfahren zur Herstellung von Verbindungen der Formel (I) 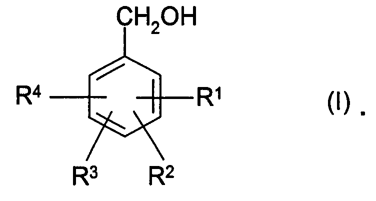 Es ist dadurch gekennzeichnet, daß man eine Verbindung der Formel (II) 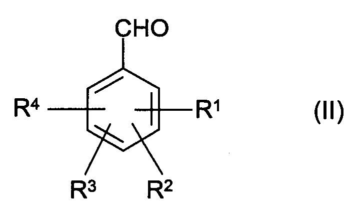 in Gegenwart eines Palladium oder Platin enthaltenden Katalysators, in Anwesenheit oder Abwesenheit eines Lösungsmittels mit Wasserstoff bei 0 bis 120°C umsetzt, wobei die Reste R¹, R², R³ und R⁴ in der Formel (I) dieselbe Bedeutung wie in Formel (II) haben und R¹ für Cl oder F steht, R², R³ und R⁴ unabhängig voneinander gleich oder verschieden sind und für H, F, C₁, CF₃, OCF₃, C₁-C₄-Alkyl oder C₁-C₄-Alkoxy stehen.

Palladium und Platin enthaltende Katalysatoren stellen in Verbindung mit Wasserstoff recht aktive und wirkungsvolle Hydrierkatalysatoren dar. Halogenierte aromatische Verbindungen, insbesondere solche, die durch elektronenziehende Substituenten wie -NO₂, -CHO oder -CH₂OH aktiviert sind, neigen unter Hydrierbedingungen zur Dehalogenierung. Dabei wird das Halogen am aromatischen Kern durch Wasserstoff ersetzt und zugleich entsteht Halogenwasserstoff.

Im Hinblick hierauf ist es als überraschend anzusehen, daß das erfindungsgemäße Verfahren trotz Verwendung von recht aktiven und wirkungsvollen Hydrierkatalysatoren die Herstellung halogenierter Benzylalkohole durch katalytische Hydrierung entsprechend halogenierter Aldehyde ermöglicht und die gewünschten halogenierten Benzylalkohole in großer Ausbeute und zugleich hoher Reinheit liefert. Es muß als unerwartet bewertet werden, daß durch eine elektronenziehende Aldehydgruppe aktivierte aromatische Verbindungen nicht oder nur in überraschend geringem Umfange unter Abspaltung von Halogenwasserstoff zu den entsprechenden dehalogenierten Verbindungen, sondern zu den entsprechenden halogenierten Benzylalkoholen umgesetzt werden.

Es ist ferner überraschend, daß es bei der Hydrierung nicht oder nur in geringem Umfang zur Bildung von weiteren Nebenprodukten, Folgeprodukten, beispielsweise unter Wasserabspaltung (Entstehung entsprechender Toluolderivate oder dimerer Produkte) oder Reaktionsprodukten der Verbindungen der Formel (I) und (II) miteinander kommt, wie sie üblicherweise bei Hydrierungen auftreten.

Die erfindungsgemäße Umsetzung verläuft - im verkürzter, vereinfachter Form wiedergegeben - nach der folgenden Reaktionsgleichung:

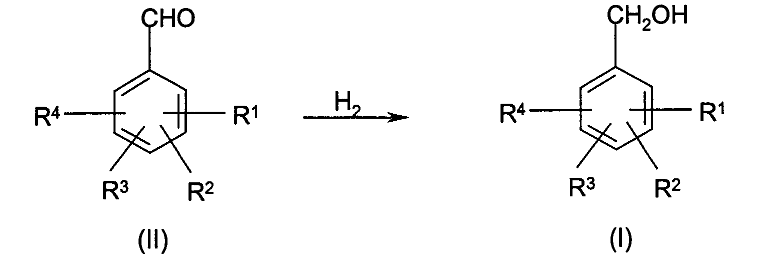

Als Wasserstoff kann Wasserstoffgas oder in situ hergestellter Wasserstoff dienen, der beispielsweise aus einer Verbindung, die unter den erfindungsgemäßen Reaktionsbedingungen Wasserstoff abspaltet, freigesetzt wird. Derartige Verbindungen, die im Sinne einer Transfer-Hydrierung Wasserstoff liefern, sind, ohne Anspruch auf Vollständigkeit zu erheben, beispielsweise Cyclohexadien, Tetralin und Ameisensäure.

Besonders einfach gestaltet sich das erfindungsgemäße Verfahren, indem man Wasserstoffgas als Wasserstoff einsetzt.

Die Reste R¹, R², R³ und R⁴ haben in Formel (II) dieselbe Bedeutung wie in Formel (I) und umgekehrt.

Man setzt als Benzaldehyd eine Verbindung der Formel (II) ein. In Formel (II) stehtwie eingangs erwähnt - R¹ für Cl oder F, insbesondere F, und R², R³ und R⁴ sind unabhängig voneinander gleich oder verschieden und stehen für H, F, Cl, CF₃, OCF₃, C₁-C₄-Alkyl oder C₁-C₄-Alkoxy, insbesondere für H, F, Cl, CF₃ oder C₁-C₄-Alkyl, bevorzugt für H, F, Cl oder C₁-C₄-Alkyl. Der Alkyl- und Alkoxyrest ist geradkettig oder verzweigt, insbesondere geradkettig.

Von Bedeutung sind Verbindungen der Formel (II), worin R² für F oder Cl, insbesondere F steht.

Von Interesse sind Verbindungen der Formel (II), worin R³ und R⁴ unabhängig voneinander gleich oder verschieden sind und für H, F, Cl oder C₁-C₄-Alkyl, insbesondere H, F, Cl oder C₁-C₄-Alkyl, bevorzugt für H oder F stehen.

Mit gutem Erfolg lassen sich Aldehyde der Formel II, worin einer der Reste R¹ und R² für F und der andere für F oder Cl steht oder beide Reste R¹ und R² für F stehen und R³ und R⁴ unabhängig voneinander gleich oder verschieden, insbesondere gleich sind und für H oder F stehen, einsetzen.

Ohne einen Anspruch auf Vollständigkeit zu erheben seien als Beispiele für Verbindungen der Formel (II) 2-Fluorbenzaldehyd, 3-Fluorbenzaldehyd, 4-Fluorbenzaldehyd, 2-Chlor-6-fluorbenzaldehyd, 2,6-Difluorbenzaldehyd, 2-Chlor-4-fluorbenzaldehyd, 2-Fluor-4-chlorbenzaldehyd, 2,4-Difluorbenzaldehyd, 2-Fluor-3-chlorbenzaldehyd, 2-Fluor-5-chlorbenzaldehyd, 2,4-Difluor-3-chlorbenzaldehyd, 2,4-Difluor-5-chlorbenzaldehyd, 4-Fluor-3,5-dichlorbenzaldehyd, 2,4,6-Trifluorbenzaldehyd, insbesondere 2-Chlor-6-fluorbenzaldehyd, 2,6-Difluorbenzaldehyd, 2,4-Difluorbenzaldehyd bevorzugt 2-Chlor-6-fluorbenzaldehyd und 2,6-Difluorbenzaldehyd genannt.

Setzt man eine Verbindung der Formel (II), worin einer oder mehrerer der Reste R¹, R², R³ und R⁴ für Cl steht, mit Wasserstoff um, so empfiehlt es sich, einen Platin enthaltenden Katalysator zu verwenden. Läßt man eine Verbindung der Formel (II), worin keiner der Reste R¹, R², R³ und R⁴ für Cl steht, mit Wasserstoff reagieren, so kann man sowohl einen Palladium enthaltenden Katalysator als auch einen Platin enthaltenden Katalysator verwenden.

Wie vorstehend erwähnt, setzt man einen Palladium oder Platin enthaltenden Katalysator, insbesondere einen Palladium oder Platin enthaltenden Trägerkatalysator ein. Der Palladium - respektive Platin-Trägerkatalysator läßt sich nach erfolgter Umsetzung beispielsweise durch Dekantieren oder Filtrieren abtrennen. Dadurch gestaltet sich das Verfahren besonders einfach. Der abgetrennte Katalysator kann wieder in die Reaktion eingesetzt werden. Es ist auch möglich, den Palladium- oder Platin-Trägerkatalysator als Festbett, beispielsweise in einem entsprechenden Reaktor, anzuordnen und die Hydrierung im Gleichstrom oder Gegenstrom durchzuführen, wobei der Aldehyd der Formel (II), in Anwesenheit oder Abwesenheit des Lösungsmittels, und der Wasserstoff in der gleichen Richtung oder einander entgegengesetzt strömen. Im Gegenstromverfahren führt man üblicherweise den Aldehyd in Anwesenheit oder Abwesenheit des Lösungsmittels von oben nach unten und schickt den Wasserstoff von unten nach oben durch den den Palladium- oder Platin-Katalysator als Festbett enthaltenden Reaktor. Die Verwendung eines Festbettes eignet sich besonders für eine kontinuierliche Arbeitsweise.

Man setzt üblicherweise einen Aktivkohle, Al₂O₃, SiO₂, Kieselgel oder Kieselgur, insbesondere Aktivkohle, Al₂O₃ oder Kieselgur, bevorzugt Aktivkohle oder Al₂O₃ als Träger enthaltenden Palladium- oder Platin-Trägerkatalysator ein.

In einer Reihe von Fällen hat sich die Verwendung eines Palladium-Aktivkohle-Katalysators oder Platin-Aktivkohle-Katalysators als günstig erwiesen.

Die Trägerkatalysatoren enthalten üblicherweise 0,05 bis 15, insbesondere 0,1 bis 10, bevorzugt 0,2 bis 8, besonders bevorzugt 0,5 bis 6 Gew.-% Pd oder Pt, bezogen auf den Trägerkatalysator.
Man setzt üblicherweise den Katalysator entsprechend 0,0001 bis 0,1, insbesondere 0,0002 bis 0,002, bevorzugt 0,0004 bis 0,0015 mol Pd oder Pt je mol Verbindung der Formel (II) ein.

Das erfindungsgemäße Verfahren läßt sich bei Einsatz von Aldehyden der Formel (II), die unter den angewendeten Reaktionsbedingungen flüssig sind, besonders einfach in Abwesenheit eines Lösungsmittels durchführen. Es ist allerdings auch möglich, bei diesen Aldehyden in Anwesenheit eines Lösungsmittels zu arbeiten.

Verwendet man einen Aldehyd (Verbindung der Formel (II)), der unter den angewendeten Reaktionsbedingungen nicht flüssig ist, so empfiehlt es sich, die Umsetzung in Anwesenheit eines Lösungsmittels durchzuführen. Als Lösungsmittel kommen insbesondere solche in Betracht, die sich unter den angewendeten Reaktionsbedingungen inert verhalten.

Man kann als Lösungsmittel einen aliphatischen, cycloaliphatischen oder aromatischen Kohlenwasserstoff, einen aliphatischen Alkohol mit 1 bis 6 Kohlenstoffatomen, ein aliphatisches oder cycloaliphatisches Keton mit 3 bis 8 Kohlenstoffatomen, einen Dialkylether mit 2 bis 5 Kohlenstoffatomen je Alkylrest, einen cyclischen Ether, einen Ester einer aliphatischen Carbonsäure mit 1 bis 6 Kohlenstoffatomen und eines aliphatischen Alkohols mit 1 bis 4 Kohlenstoffatomen oder ein Gemisch der vorstehenden Lösungsmittel einsetzen, insbesondere einen aromatischen Kohlenwasserstoff mit 6 bis 9, insbesondere 7 bis 9 Kohlenstoffatomen, einen aliphatischen Alkohol mit 1 bis 6 Kohlenstoffatomen, ein aliphatisches Keton mit 3 bis 8 Kohlenstoffatomen, einen cyclischen Ether mit 4 oder 5 C-Atomen im Ring, einen Ester einer aliphatischen Carbonsäure mit 1 bis 6, insbesondere 2 bis 4 Kohlenstoffatomen, und eines aliphatischen Alkohols mit 1 bis 4, insbesondere 1 bis 2 Kohlenstoffatomen, oder ein Gemisch der vorstehenden Lösungsmittel einsetzen.

Ohne Anspruch auf Vollständigkeit seien als Beispiele für Lösungsmittel n-Pentan, n-Hexan, Cyclopentan, Cyclohexan, Methylcyclohexan, Toluol, o-Xylol, m-Xylol, p-Xylol, technische Gemische isomerer Xylole, Ethylbenzol, Mesitylen, Methanol, Ethanol, n-Propanol, i-Propanol, n-Butanol, i-Butanol, n-Pentanol, 2-Methylpentanol, 3-Methylpentanol, n-Hexanol, Aceton, Methylethylketon, Methylisobutylketon, Diethylether, Dipropylether, Di-n-butylether, Methyl-tert.-butylether, Tetrahydrofuran, Dioxan, Essigsäuremethylester, Essigsäureethylester, Essigsäurebutylester, Propionsäuremethylester, Propionsäureethylester, Buttersäuremethylester, Buttersäureethylester genannt.
Gut geeignet als Lösungsmittel sind Toluol, o-Xylol, m-Xylol, p-Xylol, technische Gemische isomerer Xylole, Methanol, Ethanol, n-Propanol, i-Propanol, n-Butanol, i-Butanol, Aceton, Methylethylketon, Methylisobutylketon, Tetrahydrofuran, Essigsäureethylester, Essigsäurebutylester, insbesondere Toluol, o-Xylol, m-Xylol, p-Xylol, technische Gemische isomerer Xylole, Methanol, i-Propanol, Aceton, Methylethylketon, Methylbutylketon, Essigsäureethylester und Essigsäurebutylester.

Nach einer besonderen Verfahrensvariante kann man als Lösungsmittel Wasser oder ein Gemisch aus Wasser und einem oder mehreren der vorstehend genannten Lösungsmittel einsetzen. Die Anwesenheit von Wasser wirkt sich in einer Reihe von Fällen günstig aus und kann zu einer Verbesserung der Selektivität der Umsetzung und Herabsetzung der Bildung von Nebenprodukten führen. Man kann also Wasser anstelle eines der vorstehend genannten Lösungsmittel oder ein Gemisch aus Wasser mit einem der vorstehend genannten Lösungsmittel verwenden. Verwendet man ein Gemisch, so ist es günstig, ein mit Wasser mischbares oder Wasser gut lösendes Lösungsmittel, beispielsweise Aceton, Tetrahydrofuran, Isopropanol, zu benutzen. Die Mischungen enthalten üblicherweise 0,5 bis 95, insbesondere 5 bis 90, bevorzugt 20 bis 60 Gew.-% Wasser und 99,5 bis 5, insbesondere 95 bis 10, bevorzugt 80 bis 40 Gew.-% eines der vorstehend genannten organischen Lösungsmittel oder Lösungsmittelgemische.

Man legt üblicherweise die Verbindung der Formel (II), gegebenenfalls das Lösungsmittel oder Lösungsmittelgemisch und den Palladium oder Platin enthaltenden Katalysator vor, inertisiert durch Zugabe eines Inertgases, beispielsweise Stickstoff oder Argon, den Reaktor und stellt durch Aufpressen von Wasserstoff den gewünschten Druck ein.

Man führt die Umsetzung in der Regel unter einem Druck von 0,1 bis 50, insbesondere 0,5 bis 15, bevorzugt 1 bis 6 MPa durch. Für eine gute Durchmischung, beispielsweise durch Rühren, ist zu sorgen. Da während der Umsetzung Wasserstoff verbraucht wird, stellt man durch Aufpressen von Wasserstoff während der Umsetzung den gewünschten Druck ein. Das Ende der Umsetzung zeigt sich, da kein Wasserstoff mehr verbraucht wird, durch Druckkonstanz an. Diese Arbeitsweise eignet sich insbesondere für eine diskontinuierliche Umsetzung.

Es ist auch möglich, das Lösungsmittel oder Lösungsmittelgemisch und den Palladium oder Platin enthaltenden Katalysator vorzulegen, den Reaktor durch Zugabe des Inertgases zu inertisieren, Wasserstoff aufzupressen und das Ausgangsmaterial (Verbindung der Formel (II)) in reiner Form oder gelöst in dem Lösungsmittel oder Lösungsmittelgemisch zuzudosieren.

Eine kontinuierliche Arbeitsweise läßt sich- wie zuvor bereits erläutert-beispielsweise mit einem als Festbett angeordneten Katalysator in einem geeigneten Druckreaktor technisch durchführen. Der Aldehyd der Formel (II) und gegebenenfalls das Lösungsmittel oder Lösungsmittelgemisch einerseits und der Wasserstoff andererseits können im Gleichstrom oder im Gegenstrom über den als Festbett angeordneten Katalysator geführt werden.

Während bei der diskontinuierlichen Arbeitsweise der Katalysator aus dem Reaktionsgemisch, beispielsweise durch Dekantieren oder Filtrieren, abgetrennt wird, entfällt diese Abtrennung bei der kontinuierlichen Verfahrensführung, da der Palladium- oder Platin enthaltende Katalysator im Festbett verbleibt und somit nicht in das Reaktionsgemisch gelangt.

Man kann die Umsetzung - wie zuvor erwähnt bei einer Temperatur von 0 bis 120, insbesondere 10 bis 80°C, bevorzugt 15 bis 60°C durchführen. In vielen Fällen hat sich eine Temperatur von 20 bis 50°C als ausreichend erwiesen. Niedrige Temperaturen führen zu einem langsameren Reaktionsverlauf, während hohe Temperaturen einen rascheren Reaktionsverlauf begünstigen. Die Wahl der Temperatur hängt auch von der Reaktivität des Aldehyds der Formel (II) und der Aktivität des verwendeten Katalysators ab. Reaktive Aldehyde und/oder aktive Katalysatoren erlauben eine Arbeitsweise bei niedrigen Temperaturen, während hohe Temperaturen bei weniger reaktiven Aldehyden und/oder weniger aktiven Palladium oder Platin enthaltenden Katalysatoren angewendet werden können.

Zur weiteren Aufarbeitung respektive Reinigung wird das Lösungsmittel und/oder Wasser - sofern vorhanden - von der vom Katalysator abgetrennten Reaktionsmischung abdestilliert. Das Wertprodukt wird durch fraktionierte Destillation oder Kristallisation aus der Reaktionsmischung abgetrennt.

Die nachfolgenden Beispiele beschreiben die Erfindung näher, ohne sie zu beschränken.

### Experimenteller Teil

### Allgemeine Verfahrensvorschrift

Die in den nachfolgenden Beispielen beschriebenen Hydrierungen werden in einem 2-Liter Edelstahlautoklaven, der mit einem Begasungsrührer bestückt ist, durchgeführt.

Man legt Aldehyd (Verbindung der Formel (II)), gegebenenfalls zusammen mit einem Lösungsmittel oder Lösungsmittelgemisch, im Autoklaven vor und setzt den Katalysator zu. Der Autoklav wird durch Ausblasen mit einem Inertgas inertisiert und anschließend mit Wasserstoff gefüllt. Die Reaktion wird durch Einschalten der Rührung gestartet. Die Rührgeschwindigkeit beträgt 1000 bis1500 rpm. Der vorgegebene Reaktionsdruck wird durch Aufpressen von Wasserstoff während der Umsetzung eingestellt. Die Reaktion verläuft leicht exotherm. Die gewünschte Reaktionstemperatur wird durch Kühlung eingestellt. Nach beendeter Umsetzung (Ende der Wasserstoffaufnahme) wird der Autoklav entspannt, die Reaktionsmischung entnommen und der Katalysator abfiltriert.

Das Lösungsmittel und/oder Wasser werden - sofern vorhanden - von der Reaktionsmischung abdestilliert. Das Wertprodukt wird durch fraktionierte Destillation aus der Reaktionsmischung abgetrennt.

Den nachfolgenden Beispielen sind Art und Menge der Einsatzprodukte (Aldehyd der Formel (II), gegebenenfalls Lösungsmittel, Katalysator) sowie die Reaktionsbedingungen (Temperatur, Druck, Reaktionsdauer) sowie der Umsatz und die Zusammensetzung des vom Katalysator abgetrennten Reaktionsprodukts (bestimmt mittels gaschromatographischer Analyse und lösungsmittelfrei gerechnet) zu entnehmen.

### Beispiel 1

### Herstellung von 2,6-Difluorbenzylalkohol durch Umsetzung von 2,6-Difluorbenzaldehyd ohne Lösungsmittel

Man setzt, wie in der allgemeinen Verfahrensvorschrift angegeben, 10 mol (1421 g) 2,6-Difluorbenzaldehyd und 28,4 g Pd/C (Palladium-Aktivkohle Katalysator) (5 Gew.-% Pd, 50 Gew.-% wasserfeucht), bei 25 - 30°C und 2 bis 3 MPa über eine Zeit von 130 Minuten um.
Der Umsatz beträgt > 99,9 %. Das Reaktionsprodukt enthält 97,4 Gew.-% 2,6-Difluorbenzylalkohol und 2,6 Gew.-% Nebenprodukte.

### Beispiel 2

### Herstellung von 2,6-Difluorbenzylalkohol durch Umsetzung von 2,6-Difluorbenzaldehyd unter Zusatz von Wasser

Man setzt, wie in der allgemeinen Verfahrensvorschrift angegeben, 5 mol (710,5 g) 2,6-Difluorbenzaldehyd, 50 g Wasser und 14,2 g Pd/C (5 Gew.-% Pd; 50 Gew.-% wasserfeucht) bei 40°C und 2 bis 3 MPa über eine Zeit von 55 Minuten um. Der Umsatz beträgt > 99,9 %. Das Reaktionsprodukt enthält 99,5 Gew.-% 2,6-Difluorbenzylalkohol und 0,4 Gew.-% Nebenprodukte.

### Beispiel 3

### Herstellung von 2,6-Difluorbenzylalkohol durch Umsetzung von 2,6-Difluorbenzaldehyd mittels eines Pt/C Katalysators unter Zusatz von Wasser

Man setzt, wie in der allgemeinen Verfahrensvorschrift angegeben, 5 mol (710,5 g) 2,6-Difluorbenzaldehyd, 50 g Wasser und 14,2 g Pt/C (Platin-Aktivkohle Katalysator) (5 Gew.-% Pt; 50 Gew.-% wasserfeucht) bei 35°C und 3,5 bis 4 MPa über eine Zeit von 50 Minuten um.
Der Umsatz beträgt 99,7 %. Das Reaktionsprodukt enthält 99,0 Gew.-% 2,6-Difluorbenzylalkohol und 0,8 Gew.-% Nebenprodukte.

### Beispiel 4

### Herstellung von 2,6-Difluorbenzylalkohol durch Umsetzung von 2,6-Difluorbenzaldehyd unter Zusatz von Aceton und Wasser

Man setzt, wie in der allgemeinen Verfahrensvorschrift angegeben, 4,6 mol (650 g) 2,6-Difluorbenzaldehyd, 395 g Aceton, 50 g Wasser und 14,2 g Pd/C (5 Gew.-% Pd; 50 Gew.-% wasserfeucht) bei 40°C und 2 bis 3 MPa über eine Zeit von 40 Minuten um.
Der Umsatz beträgt > 99,9 %. Das Reaktionsprodukt enthält 99,5 Gew.-% 2,6-Difluorbenzylalkohol und 0,5 Gew.-% Nebenprodukte.

### Beispiel 5

### Herstellung von 2,6-Difluorbenzylalkohol unter Zudosierung von 2,6-Difluorbenzaldehyd

In Abweichung von der allgemeinen Verfahrensvorschrift werden in den Autoklaven 420 g Essigsäuremethylester, 50 g Wasser und 14,2 g Pd/C (5 Gew.-% Pd; 50 Gew.-% wasserfeucht) vorgelegt, der Autoklav wird inertisiert und anschließend wird unter Rühren Wasserstoff zugesetzt. Zu der unter einen Druck von 2 bis 3 MPa stehenden Suspension werden bei 40°C 5 mol (710,5 g) 2,6-Difluorbenzaldehyd innerhalb von 70 Minuten zudosiert. Nach vollständiger Dosierung ist die Hydrierung beendet.
Der Umsatz beträgt > 99,9 %. Das Reaktionsprodukt enthält 99,9 Gew.-% 2,6-Difluorbenzylalkohol und 0,1 Gew.-% Nebenprodukte.

### Beispiel 6

### Herstellung von 2-Chlor-6-fluorbenzylalkohol durch Umsetzung von 2-Chlor-6-fluorbenzaldehyd mittels eines Pt/C-Katalysators unter Zusatz von Aceton und Wasser

Man setzt, wie in der allgemeinen Verfahrensvorschrift angegeben, 1,57 mol (250 g) 2-Chlor-6-fluorbenzaldehyd, 250 g Aceton, 25 g Wasser und 25 g Pt/C (5 Gew.-% Pt; 50 Gew.-% wasserfeucht) bei 20 bis 25°C und 1 bis 1,5 MPa über eine Zeit von 150 Minuten um.
Der Umsatz beträgt 99,3 %. Das Reaktionsprodukt enthält 91,50 Gew.-% 2-Chlor-6-fluorbenzylalkohol und 7,8 Gew.-% Nebenprodukte.

### Beispiel 7

### Herstellung von 2,6-Difluorbenzylalkohol unter Zudosierung von 2,6-Difluorbenzaldehyd bei höherer Temperatur

In Abweichung von der allgemeinen Verfahrensvorschrift werden in den Autoklaven 420 g Essigsäuremethylester, 50 g Wasser und 14,2 g Pd/C (5 Gew.-% Pd; 50 Gew.-% wasserfeucht) vorgelegt, der Autoklav wird inertisiert und anschließend wird unter Rühren Wasserstoff zugesetzt. Zu der unter einem Druck von 2 bis 3 MPa stehenden Suspension werden bei 60°C 5 mol (710,5 g) 2,6-Difluorbenzaldehyd innerhalb von 70 Minuten zudosiert.
Nach vollständiger Dosierung ist die Hydrierung beendet.
Der Umsatz beträgt > 99,9 %. Das Reaktionsprodukt enthält 98,2 Gew.-% 2,6-Difluorbenzylalkohol und 1,8 Gew.-% Nebenprodukte.

### Beispiel 8

### Herstellung von 2,6-Difluorbenzylalkohol durch Umsetzung von 2,6-Difluorbenzaldehyd mittels recyclisiertem Pd/C Katalysator

Man setzt, wie in der allgemeinen Verfahrensvorschrift angegeben, 5 mol (710,5 g) 2,6-Difluorbenzaldehyd, 50 g Wasser und 17,5 g recyclisierten Pd/C Katalysator (5 Gew.-% Pd, 60 Gew.-% wasserfeucht) bei 40°C und 2 bis 3 MPa über eine Zeit von 58 Minuten um.
Der Umsatz beträgt > 99,9 %. Das Reaktionsprodukt enthält 99,6 Gew.-% 2,6-Difluorbenzaldehyd und 0,4 Gew.-% Nebenprodukte.

## Patentansprüche

1. Verfahren zur Herstellung von Verbindungen der Formel (I) 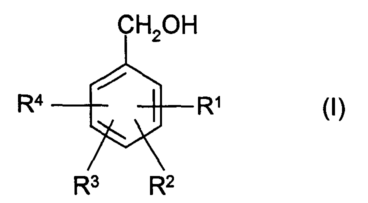 dadurch gekennzeichnet, daß man eine Verbindung der Formel (II) 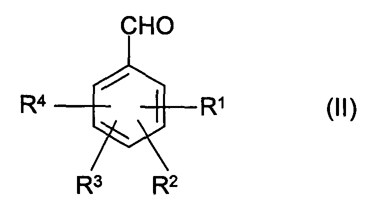 in Gegenwart eines Palladium oder Platin enthaltenden Katalysators, in Anwesenheit oder Abwesenheit eines Lösungsmittels mit Wasserstoff bei 0 bis 120°C umsetzt, wobei die Reste R¹, R², R³ und R⁴ in der Formel (I) dieselbe Bedeutung wie in Formel (II) haben und R¹ für Cl oder F steht, R², R³ und R⁴ unabhängig voneinander gleich oder verschieden sind und für H, F, Cl, CF₃, OCF₃, C₁-C₄-Alkyl oder C₁-C₄-Alkoxy stehen.

2. Verfahren nach Anspruch 1, dadurch gekennzeichnet, daß man eine Verbindung der Formel (I), worin R¹ für F steht, einsetzt.

3. Verfahren nach Anspruch 1 oder 2, dadurch gekennzeichnet, daß man eine Verbindung der Formel (II), worin R², R³ und R⁴ unabhängig voneinander gleich oder verschieden sind und für H, F, Cl, CF₃ oder C₁-C₄-Alkyl stehen, einsetzt.

4. Verfahren nach einem oder mehreren der Ansprüche 1 bis 3, dadurch gekennzeichnet, daß man eine Verbindung der Formel (II), worin R² für F oder Cl steht, einsetzt.

5. Verfahren nach einem oder mehreren der Ansprüche 1 bis 4, dadurch gekennzeichnet, daß man eine Verbindung der Formel (II), worin R³ und R⁴ unabhängig voneinander gleich oder verschieden sind und für H, F, Cl oder C₁-C₄-Alkyl stehen, einsetzt.

6. Verfahren nach einem oder mehreren der Ansprüche 1 bis 5, dadurch gekennzeichnet, daß man einen Palladium oder Platin enthaltenden Trägerkatalysator einsetzt.

7. Verfahren nach einem oder mehreren der Ansprüche 1 bis 6, dadurch gekennzeichnet, daß man einen Aktivkohle, Al₂O₃, SiO₂, Kieselgel oder Kieselgur als Träger enthaltenden Palladium- oder Platin-Trägerkatalysator einsetzt.

8. Verfahren nach einem oder mehreren der Ansprüche 1 bis 7, dadurch gekennzeichnet, daß man einen Palladium-Aktivkohle-Katalysator oder einen Platin-Aktivkohle-Katalysator einsetzt.

9. Verfahren nach einem oder mehreren der Ansprüche 1 bis 8, dadurch gekennzeichnet, daß man als Lösungsmittel einen aliphatischen, cycloaliphatischen oder aromatischen Kohlenwasserstoff, einen aliphatischen Alkohol mit 1 bis 6 Kohlenstoffatomen, ein aliphatisches oder cycloaliphatisches Keton mit 3 bis 8 Kohlenstoffatomen, einen Dialkylether mit 2 bis 5 Kohlenstoffatomen je Alkylrest, einen cyclischen Ether, einen Ester einer aliphatischen Carbonsäure mit 1 bis 6 Kohlenstoffatomen und eines aliphatischen Alkohols mit 1 bis 4 Kohlenstoffatomen oder ein Gemisch der vorstehenden Lösungsmittel einsetzt.

10. Verfahren nach einem oder mehreren der Ansprüche 1 bis 9, dadurch gekennzeichnet, daß man als Lösungsmittel Wasser oder ein Gemisch aus Wasser und einem oder mehreren der vorstehend genannten Lösungsmittel einsetzt.

11. Verfahren nach einem oder mehreren der Ansprüche 1 bis 10, dadurch gekennzeichnet, daß man die Umsetzung unter einem Druck von 0,1 bis 50 MPa durchführt.
